# EUROPÄISCHE PATENTANMELDUNG

(11) **EP 4 339 963 A1**
(43) Veröffentlichungstag der Anmeldung: **20.03.2024**
(21) Anmeldenummer: 22195490.2
(22) Anmeldetag: 14.09.2022
(51) Int. Cl.: G16H 30/00, G16H 30/20, G16H 30/40

(54) **TECHNIK ZUR SICHERHEITSKONTROLLE EINES MEDIZINISCHEN BEFUNDS**

(71) Anmelder: Siemens Healthineers AG, 91301 Forchheim (DE)
(72) Erfinder: Kohl, Gerhard, 91077 Neunkirchen am Brand (DE); Kohle, Sven, 91056 Erlangen (DE); Schaller, Volker, 91080 Uttenreuth (DE); Speier, Christoph, 91052 Erlangen (DE); Tietjen, Christian, 90763 Fürth (DE); Nolte, Björn, 90765 Fürth (DE)
(74) Vertreter: Siemens Healthineers Patent Attorneys

(57) **Zusammenfassung**

Die vorliegende Erfindung betrifft ein computer-implementiertes Verfahren zur Sicherheitskontrolle eines medizinischen Befunds für einen Patienten basierend auf einer Bilddatei, die mittels eines bildgebenden medizinischen Geräts erfasst wurde. Das Verfahren umfasst die Schritte des Einlesens (S102) von Sensordaten bezüglich der Bilddatei; Zuordnens (S104) der eingelesenen Sensordaten zu einer ersten Position einer anatomischen Struktur des Patienten; Extrahierens (S106) mindestens einer zweiten Position einer anatomischen Struktur des Patienten aus dem medizinischen Befund; Vergleichens (S108) der ersten Position und der mindestens einen zweiten Position; und des Ausgebens (S110) eines Warnsignals, falls der Vergleich (S108) keine Übereinstimmung ergibt.

## Beschreibung

Die vorliegende Erfindung betrifft ein computer-implementiertes Verfahren zur Sicherheitskontrolle eines medizinischen Befunds basierend auf einer Bilddatei, die mittels eines bildgebenden medizinischen Geräts erfasst wurde. Die Erfindung betrifft ferner eine Vorrichtung, ein System, ein Computerprogrammprodukt und ein computerlesbares Speichermedium, die jeweils zur Ausführung des Verfahrens ausgebildet sind.

In einer radiologischen Befundung werden Auffälligkeiten in Bilddaten einer aktuellen Untersuchung herkömmlicherweise in einem Befund beschrieben und dabei häufig in den aktuellen Bildern markiert oder auch vermessen. Wenn es Voruntersuchungen des Patienten der gleichen oder auch anderer Untersuchungsmodalitäten gibt, werden diese mitunter mit zum Vergleich herangezogen.

Aus medizinischer medico-legaler Sicht ist es wichtig, dass in einem Befund alle wichtigen Pathologien, die auf den gesehen Bildern erkennbar waren, beschrieben worden sind. Fehlende Informationen im Befund können zu Diagnose- und/oder Behandlungsfehlern führen. Aus diesem Grund werden beispielsweise in Deutschland die Befunde durch Oberärzte überprüft oder supervidiert.

Trotz dieser doppelten Begutachtung der Bilder und des Befunds werden Pathologien übersehen. Darüber hinaus ist es im Interesse der Erstbefunderin oder des Erstbefunders, dass der Befundtext bereits zum Zeitpunkt der Überprüfung möglichst fehlerlos und vollständig ist.

Häufig zeigen sich in einem radiologischen Bild neben dem Hauptbefund weitere Nebenbefunde. Besonders, wenn ein radiologisches Bild viele Nebenbefunde aufweist, ist es herkömmlicherweise möglich, dass die befundende Radiologin oder der befundende Radiologe vergisst, einen oder mehrere Nebenbefunde im Befundtext zu erwähnen.

Derzeit wird der Befund lediglich von einer zweiten Radiologin oder einem zweiten Radiologen händisch überprüft. Die Fehleranfälligkeit, insbesondere bei mehreren Nebenbefunden, bleibt jedoch weiterhin bestehen.

Der vorliegenden Erfindung liegt die Aufgabe zugrunde, eine Technik bereitzustellen, die sicherstellt, dass ein Befund alle relevanten Informationen, insbesondere hinsichtlich aller betrachteten Bildbereiche, und/oder sowohl den Hauptbefund als auch alle Nebenbefunde enthält. Alternativ oder ergänzend besteht die Aufgabe, die Auswertung einer Bilddatei zu verbessern und somit eine Qualitätsverbesserung einer Diagnose und/oder einer gerätebasierten Behandlung und/oder Behandlungsstrategie zu ermöglichen.

Diese Aufgabe wird jeweils durch einen Gegenstand nach den unabhängigen, nebengeordneten Ansprüchen gelöst. Vorteilhafte Ausführungsformen sind Gegenstand der abhängigen Ansprüche, der Beschreibung und der Zeichnungen.

Gemäß einem Verfahrensaspekt wird ein computer-implementiertes Verfahren zur Sicherheitskontrolle eines medizinischen Befunds für einen Patienten aufgrund einer Bilddatei bereitgestellt, wobei die Bilddatei mittels eines bildgebenden medizinischen Geräts erfasst wurde. Das Verfahren umfasst den Schritt des Einlesens von Sensordaten bezüglich der Bilddatei. Das Verfahren umfasst ferner den Schritt des Zuordnens der eingelesenen Sensordaten zu einer ersten Position einer anatomischen Struktur des Patienten. Das Verfahren umfasst ferner den Schritt des Extrahierens mindestens einer zweiten Position einer anatomischen Struktur des Patienten aus dem medizinischen Befund. Das Verfahren umfasst ferner den Schritt des Vergleichens der ersten Position und der mindestens einen zweiten Position. Das Verfahren umfasst ferner den Schritt des Ausgebens eines Warnsignals, falls der Vergleich keine Übereinstimmung ergibt.

Die Sensordaten bezüglich der Bilddatei können einen Fokusbereich definieren. Der Fokusbereich kann während einer Betrachtung der Bilddatei, insbesondere der Betrachtung der ersten Position, durch geschultes medizinisches Personal, insbesondere einen Arzt, im Fokus stehen. Beispielsweise können die Sensordaten einen oder mehrere Hinweise auf die Betrachtung, insbesondere der ersten Position, durch geschultes medizinisches Personal, insbesondere einen Arzt, geben. Alternativ oder ergänzend kann das geschulte medizinische Personal, insbesondere der Arzt, Autor des medizinischen Befunds sein.

Der Hinweis, oder die Hinweise, auf die Betrachtung kann, insbesondere digital, eine oder mehrere auf der Bilddatei ausgeführte Bewegungen und/oder Eingaben mittels einer Nutzerschnittstelle umfassen. Die Bewegungen und/oder Eingaben können beispielsweise Computermaus-Bewegungen, Trackpad-Bewegungen, Trackball-Bewegungen, Tasten-Bewegungen (beispielsweise mittels Tasten einer Tastatur), Wischbewegungen auf einem berührungsempfindlichen Bildschirm und/oder Eingaben mittels der entsprechenden Eingabemittel (z.B. Computermaus, Trackpad, Trackball, Joystick, Tastatur, und/oder berührungsempfindlicher Bildschirm) umfassen. Beispielsweise können die Bewegungen und/oder Eingaben ein Zoomen in einen Bereich, der auch als Fokusbereich bezeichnet werden kann, umfassen.

Alternativ oder ergänzend können die Eingaben beispielsweise Messungen (z.B. Distanzmessungen und/oder Größenmessungen eines Organs) auf der Bilddatei und/oder Annotationen (z.B. Markierungen mittels einfacher graphischer Symbole und/oder kurze Texte) auf der Bilddatei umfassen.

Alternativ oder ergänzend kann der Hinweis, oder die Hinweise, auf die Betrachtung mittels Augen-Verfolgung (englisch: eye tracking) bestimmt werden.

Weiterhin alternativ oder ergänzend kann die Bilddatei einen Bildstapel von Schichten umfassen. Eine Hitzekarte (englisch: heatmap) kann eine Dauer der Betrachtung einzelner oder aller Schichten des Bildstapels umfassen. Beispielsweise kann eine lange (und damit intensiv) betrachtete Schicht oder ein lange /intensiv analysierter Bereich im Bild einem Fokusbereich zugeordnet werden.

Das Einlesen von Sensordaten bezüglich der Bilddatei kann zur Bestimmung des Fokusbereichs geeignet sein. Alternativ oder ergänzend kann das Einlesen der Sensordaten bezüglich der Bilddatei die Bestimmung des Fokusbereichs umfassen.

Der Fokusbereich kann einem Bereich der Bilddatei entsprechen, der im Fokus der Betrachtung durch das geschulte medizinische Personal, insbesondere den Arzt, stand und/oder für den es einen oder mehrere Hinweise auf die Betrachtung durch das geschulte medizinische Personal, insbesondere den Arzt, gibt.

Der Fokusbereich kann die erste Position der anatomischen Struktur des Patienten umfassen und/oder ihr zugeordnet sein. Die erste Position der anatomischen Struktur des Patienten kann eine gesunde Struktur, einen verdächtigen Bereich, und/oder eine Läsion (auch: abnormale Struktur oder kranke Struktur) umfassen oder identifizieren. Beispielsweise kann der Fokusbereich einem Bereich der Bilddatei entsprechen, der eine möglicherweise anomale Gewebestruktur oder eine Läsion des Patienten zeigt. Eine Gewebestruktur kann möglicherweise als Läsion eingestuft werden aufgrund einer Farbe und/oder einer Transparenz, einer Opazität und/oder einer Form in einem Bereich der Bilddatei, insbesondere (an) der ersten Position.

Der ersten Position kann ein Text und/oder eine Kodierung zugeordnet werden.

Der Befund kann auch als Arztbericht bezeichnet werden. Der Befund kann eine Bewertung der Bilddatei durch das geschulte medizinische Personal, insbesondere den Arzt, hinsichtlich der Einstufung als gesunde Strukturen, verdächtige Bereiche, und/oder Läsionen umfassen.

Der Befund kann einen Fließtext und/oder eine Kodierung umfassen. Die Kodierung kann die mindestens eine zweite Position umfassen. Alternativ oder ergänzend kann die Kodierung eine Beschreibung und/oder eine Klassifizierung der mindestens einen zweiten Position umfassen.

Die mindestens eine zweite Position kann aus dem Fließtext und/oder aus der Kodierung extrahiert werden.

Der Vergleich der ersten Position und der mindestens einen zweiten Position kann eine Übereinstimmung ergeben. Beispielsweise kann das geschulte medizinische Personal, insbesondere der Arzt, eine erste Position als Fokusbereich der Bilddatei betrachtet, vermessen und/oder annotiert haben. Das geschulte medizinische Personal, insbesondere der Arzt kann diesen Fokusbereich in dem Befund erwähnt haben. Die Erwähnung des Fokusbereichs im Befund kann als Erwähnen der zweiten Position bezeichnet werden.

Die eingelesenen Sensordaten können zu einer Zuordnung einer Mehrzahl erster Positionen der anatomischen Struktur des Patienten führen. Das computer-implementierte Verfahren und/oder der Vergleich mit der mindestens einen zweiten Position der anatomischen Struktur des Patienten kann für jede der Mehrzahl der ersten Positionen durchgeführt werden. Alternativ oder ergänzend können die erste Position und die mindestens eine zweite Position nicht (beispielsweise nicht hinreichend) übereinstimmen. Wenn der Vergleich eine nicht (beispielsweise nicht hinreichende) Übereinstimmung ergibt, kann ein Warnsignal ausgegeben werden. Als Übereinstimmung wird insbesondere nicht zwangsläufig eine Identität der Positionen verstanden. Beispielsweise kann die Übereinstimmung basierend auf einem Übereinstimmungsmaß ermittelt werden, wobei das Übereinstimmungsmaß einen absoluten oder relativen Abstand zwischen der ersten und der mindestens einen zweiten Position beschreiben kann, zum Beispiel einen Abstand in cm oder Pixel. Als Übereinstimmung wird beispielsweise ein Übereinstimmungsmaß und/oder Abstand kleiner als ein Schwellenwert verstanden. Der Schwellenwert kann ein absoluter Schwellenwert, ein positionsabhängiger Schwellenwert oder ein Organspezifischer Schwellenwert sein.

Das Warnsignal kann einen optischen Hinweis auf einem Bildschirm umfassen, beispielsweise eine Fehlermeldung beim Verlassen eines Bearbeitungsprogramms (auch: Datenverarbeitungsprogramm) für den Befund, beim Versuch, den Befund abzuzeichnen, und/oder aufgrund eines anderweitigen Ereignisses.

Alternativ oder ergänzend kann das Verfahren zur Kontrolle durch den Autor des Befunds und/oder durch eine Kontrollperson (beispielsweise im Zuge einer Qualitätskontrolle durch einen zweiten Arzt und/oder durch eine Qualitätskontrollstelle einer medizinischen Einrichtung, der das bildgebende medizinische Gerät und/oder die Bilddatei zugeordnet ist) initiiert werden, beispielsweise mittels eines Buttons auf einer Eingabefläche.

Alternativ oder ergänzend kann das Verfahren zur Qualitätskontrolle automatisch gestartet werden, beispielsweise zu einem beliebigen und/oder einem vorbestimmten Zeitpunkt, nachdem der Autor den Befund signiert hat.

Weiterhin alternativ oder ergänzend kann das Warnsignal kontinuierlich, insbesondere während des Verfassens des Befunds, ausgegeben werden, bis die (oder jede) erste Position mit einer (beispielsweise einer jeweiligen) entsprechenden zweiten Position im Befund übereinstimmt.

Das Warnsignal und/oder der optische Hinweis auf dem Bildschirm kann eine Fehlermeldung, insbesondere im Textformat, umfassen. Alternativ oder ergänzend kann der Hinweis auf dem Bildschirm eine Wiedergabe der Bilddatei umfassen mit einer (beispielsweise roten) Markierung der (oder jeder) ersten Position, deren Vergleich mit den zweiten Positionen im Befund keine Übereinstimmung ergibt.

Alternativ oder ergänzend kann auf dem Bildschirm kontinuierlich eine Wiedergabe der Bilddatei erfolgen mit einer (beispielsweise grünen) Markierung der (oder jeder) ersten Position, für die der Vergleich mit den zweiten Positionen im Befund eine Übereinstimmung ergibt.

Insbesondere können Übereinstimmungen in einer ersten Kodierung, z.B. einer Farbe, und/oder mittels eines ersten optischen Hinweises, auf einer Wiedergabe markiert werden. Fehlende Übereinstimmungen können in einer zweiten Kodierung, insbesondere Farbe, und/oder mittels eines zweiten optischen Hinweises, auf der Widergabe markiert werden. Die erste Kodierung, insbesondere Farbe, und die zweite Kodierung, insbesondere Farbe, können unterschiedlich sein, beispielsweise grün bzw. rot. Alternativ oder ergänzend können der erste optische Hinweis und der zweite optische Hinweis unterschiedlich (z.B. hinsichtlich einer Form) sein, beispielsweise Kontouren mit verschiedenen Linienarten (z.B. durchgezogen vs. gestrichelt, oder umgekehrt) umfassen.

Alternativ oder ergänzend kann das Warnsignal ein akustisches Signal umfassen, das beispielsweise bei vorbestimmten Handlungen des geschulten medizinischen Personals, insbesondere des Arztes, ausgegeben wird. Beispielsweise kann beim (z.B. zwischenzeitlichen) Schließen des Bearbeitungsprogramms, mit dem der Befund erstellt und/oder bearbeitet wird, und/oder beim Versuch des Signierens des Befunds ein akustisches Warnsignal ausgegeben werden.

Mittels des Verfahrens, insbesondere durch den Vergleich der Sensordaten und der Erwähnungen im Befund von Positionen, kann sichergestellt werden, dass der Befund umfassend ist und/oder alle potentiell relevanten Positionen erwähnt. Alternativ oder ergänzend kann durch das Verfahren eine Qualität in der Diagnose aufgrund bzw. auf Basis der Bilddatei verbessert werden. Eine verbesserte Diagnose ermöglicht wiederum eine verbesserte Behandlungsstrategie (auch: Therapiestrategie) .

Die Sensordaten können eine Zeitdauer einer Darstellung, insbesondere auf einem Bildschirm, z.B. einer Bildschicht innerhalb eines Bildstapels medizinischer Bilder der Bilddatei umfassen. Alternativ oder ergänzend können die Sensordaten Bewegungsdaten einer Eingabeeinrichtung, insbesondere einer Computermaus, auf einer Darstellung eines Bildes oder einer Bildschicht der Bilddatei umfassen. Weiterhin alternativ oder ergänzend können die Sensordaten Bewegungsdaten einer Blickerfassung auf einer Darstellung eines Bildes oder einer Bildschicht der Bilddatei umfassen. Weiterhin alternativ oder ergänzend können die Sensordaten Markierungen eines Bereichs von Interesse auf einer Darstellung eines Bildes oder einer Bildschicht der Bilddatei umfassen. Weiterhin alternativ oder ergänzend können die Sensordaten Messungen einer Distanz, eines Durchmessers und/oder einer Dichte auf einer Darstellung eines Bildes oder einer Bildschicht der Bilddatei umfassen. Weiterhin alternativ oder ergänzend können die Sensordaten Annotationen zu einer Darstellung eines Bildes oder einer Bildschicht der Bilddatei umfassen. Weiterhin alternativ oder ergänzend können die Sensordaten Bedienungsdaten hinsichtlich einer Darstellung mindestens eines Bildes der Bilddatei umfassen.

Die Bewegungsdaten können auch als Bewegungsmuster und/oder Bedienungsmuster bezeichnet werden.

Eine Sammlung (und/oder Anordnung) von Zeitdauern der Darstellungen (beispielsweise auf einem Bildschirm zur Ansicht durch das geschulte medizinische Personal, insbesondere den Arzt) kann als Hitzekarte (englisch: heatmap) der Bildschichten des Bildstapels bezeichnet und/oder angezeigt werden.

Die Blickerfassung kann auch als "eye tracking" bezeichnet werden.

Die Bedienungsdaten der Darstellung des mindestens einen Bilds der Bilddatei können ein Zoomen auf einen Teilbereich des Bildes, insbesondere umfassend die erste Position, umfassen.

Das Zuordnen der eingelesenen Sensordaten zu der ersten Position der anatomischen Struktur des Patienten kann ein Identifizieren von anatomischen Landmarken, eine Segmentierung, insbesondere mindestens eines Organs des Patienten umfassen. Alternativ oder ergänzend kann das Zuordnen mittels einem durch maschinelles Lernen trainierten Algorithmus ausgeführt werden.

Die anatomische Landmarke kann eine auffällige und/oder prominente anatomische Struktur umfassen, beispielsweise die Wirbelsäule und/oder Teile des Knochengerüsts des Patienten. Die Segmentierung kann eine Segmentierung eines Organs oder eines anatomischen Teilbereichs, beispielsweise der Lunge, Leber oder einer Niere, umfassen.

Der mittels Maschinellem Lernen trainierte Algorithmus kann eine künstliche Intelligenz (KI; englisch: "Artificial Intelligence", kurz: AI) umfassen. Alternativ oder ergänzend kann das Maschinelle Lernen ein oder mehrere Neuronale Netzwerke umfassen, insbesondere ein Convolutional Neural Network (CNN) .

Der Algorithmus kann mittels annotierter Bilddateien, insbesondere erhalten von dem bildgebenden medizinischen Gerät oder einem Gerät derselben Art, trainiert werden. Alternativ oder ergänzend kann der trainierte Algorithmus die erste Position ohne Zwischenschritte, insbesondere eine Identifizierung von anatomischen Landmarken und/oder ohne eine Segmentierung, identifizieren.

Das Zuordnen der eingelesenen Sensordaten zu der ersten Position der anatomischen Struktur des Patienten kann einen Schritt des Ausgebens des Ergebnisses des Zuordnens in einer Kodierung umfassen. Optional kann die Kodierung Snomed und/oder RadLex umfassen.

Snomed kann als eine Computer-verarbeitbare Ontologie bzw. Sammlung von medizinischen Begriffen, Codes und damit als ein Standard von Gesundheitsbegriffen oder systematisierte Nomenklatur (auch: Terminologie im Gesundheitswesen) verstanden werden. Snomed kann unterschiedliche Unterteilungen, insbesondere Snomed-CT (Clinical Terms), u.a. auch für bildgebende medizinische Verfahren, umfassen.

RadLex kann eine standardisierte bzw. kontrollierte Terminologie für die Radiologie verstanden werden. RadLex kann andere Lexika und/oder Standards vereinheitlichen und/oder ergänzen, beispielsweise Snomed-CT, LOINC und/oder DICOM. In RadLex können neben radiologischen Begriffen auch Abhängigkeiten und Relationen der Begriffe zueinander definiert sein.

Alternativ oder ergänzend kann die Kodierung LOINC und/oder DICOM umfassen.

Das Extrahieren der mindestens einen zweiten Position der anatomischen Struktur des Patienten aus dem medizinischen Befund kann eine linguistische Datenverarbeitung eines Textes des medizinischen Befunds und eine anschließende Kodierung der aus dem linguistisch datenverarbeiteten Text extrahierten mindestens einen zweiten Position umfassen. Optional kann die Kodierung Snomed und/oder RadLex umfassen.

Das Extrahieren der mindestens einen zweiten Position der anatomischen Struktur des Patienten aus dem medizinischen Befund kann ein Ausgeben in einer Kodierung umfassen. Optional kann die Kodierung Snomed und/oder RadLex umfassen.

Das Vergleichen der den eingelesenen Sensordaten zugeordneten ersten Position mit der mindestens einen aus dem medizinischen Befund extrahierten zweiten Position kann einen computer-basierten bzw. algorithmisch ausgeführten Vergleich der jeweiligen Kodierungen umfassen. Optional kann die jeweilige Kodierung Snomed und/oder RadLex umfassen.

Das Warnsignal kann eine Ausgabe der den eingelesenen Sensordaten zugeordneten ersten Position, für die im Schritt des Vergleichens keine entsprechende aus dem medizinischen Befund extrahierte zweiten Position gefunden wurde, auf einer Darstellung eines Bildes oder einer Schicht einer 3D-Bilddatei umfassen. Alternativ oder ergänzend kann das Warnsignal eine Textausgabe einer fehlenden Übereinstimmung hinsichtlich der den eingelesenen Sensordaten zugeordneten ersten Position umfassen. Weiterhin alternativ oder ergänzend kann das Warnsignal eine Blockierung eines Arbeitsschritts des Abschließens und/oder Unterzeichnens des medizinischen Befunds umfassen. Die Blockierung des Arbeitsschritts des Abschließens und/oder Unterzeichnens des medizinischen Befunds kann von einer Bewertung einer Wichtigkeit der fehlenden Übereinstimmung abhängen. Beispielsweise kann die Wichtigkeit hoch sein, wenn die Sensordaten darauf hindeuten, dass die erste Position intensiv von dem geschulten medizinischen Personal, insbesondere dem Arzt, betrachtet, vermessen und/oder annotiert wurde. Die Wichtigkeit der fehlenden Übereinstimmung kann gering sein, wenn die Sensordaten darauf hindeuten, dass die erste Position nur sehr kurz betrachtet, nicht vermessen und/oder nicht annotiert wurde.

Das Warnsignal kann je nach Ausführungsbeispiel zu unterschiedlichen Zeitpunkten der Erstellung des Befunds ausgegeben werden.

Beispielsweise kann das Warnsignal kontinuierlich, und/oder aufgrund einer Kontrollanfrage des Autors des Befunds, während des Erstellens des Befunds ausgegeben werden.

Alternativ oder ergänzend kann das Warnsignal bei einem (z.B. zwischenzeitlichen) Schließen des Bearbeitungsprogramms des Befunds und/oder bei einem Versuch, den Befund abzuschließen und/oder zu signieren ausgegeben werden.

Alternativ oder kumulativ kann das Warnsignal bei einer Qualitätskontrolle des bereits abgeschlossenen und/oder signierten Befunds erzeugt werden.

Beispielsweise kann die Qualitätskontrolle außerhalb des Lese- und Berichts-Arbeitsablaufs des geschulten medizinischen Personals, insbesondere des Arztes, zentral auf einer Maschine, beispielsweise einem Server, eines Krankenhaus-Informations-Systems (KIS) und/oder eines Radiologie-Informations-Systems (RIS) durchgeführt werden.

Die Ausgabe des Warnsignals kann eine Aufforderung zur Korrektur und/oder Änderung des medizinischen Befunds umfassen. Damit kann die Qualität der Befundung instantan verbessert werden.

Das medizinische bildgebende Gerät kann ein Röntgengerät umfassend. Alternativ oder ergänzend kann das medizinische bildgebende Gerät einen Magnetresonanztomographen (MRT) umfassen. Weiterhin alternativ oder ergänzend kann das medizinische bildgebende Gerät einen Positronen-Emissions-Tomographen (PET) umfassen. Weiterhin alternativ oder ergänzend kann das medizinische bildgebende Gerät einen Angiographen (AX) umfassen. Weiterhin alternativ oder ergänzend kann das medizinische bildgebende Gerät einen Computertomographen (CT) umfassen. Weiterhin alternativ oder ergänzend kann das medizinische bildgebende Gerät ein Ultraschallgerät umfassen. Das Verfahren kann ferner einen Schritt des Einlesens von weiteren Patientendaten umfassen, wobei die weiteren Patientendaten unabhängig von der Bilddatei erhoben worden sind. Das Verfahren kann ferner einen Schritt des Extrahierens mindestens einer dritten Position einer anatomischen Struktur des Patienten aus den eingelesen weiteren Patientendaten umfassen.

Das Verfahren kann ferner einen Schritt des Vergleichens der aus den weiteren Patientendaten extrahierten mindestens einen dritten Position mit der ersten Position und/oder mit der mindestens einen zweiten Position umfassen.

Das Verfahren kann ferner einen Schritt des Ausgebens eines Hinweises, insbesondere in Form eines Hinweissignals, umfassen, falls der Vergleich eine Übereinstimmung ergibt, wobei der Hinweis gerichtet ist auf eine Überprüfung einer Konsistenz des Befunds mit den eingelesenen weiteren Patientendaten.

Durch das Einlesen der weiteren Patientendaten, Extrahieren einer oder mehrerer Positionen in den eingelesenen weiteren Patientendaten und Vergleichen mit der ersten Position aus den Sensordaten und/oder mit der zweiten Positionen aus dem Befund kann eine medizinische Vorgeschichte des Patienten zur Festlegung einer Diagnose hinzugezogen werden. Dadurch kann die Qualität der medizinischen Diagnose und/oder des Befunds weiter verbessert werden.

Mittels des Hinweises kann geschultes medizinisches Personal, insbesondere ein Arzt, darauf aufmerksam gemacht werden, dass eine medizinische Vorgeschichte zu der ersten Position der anatomischen Struktur existiert.

Das Verfahren kann ferner einen Schritt des Einlesens einer medizinischen Richtlinie (die auch als medizinische Leitlinie und/oder Standard of Operation, SOP, bezeichnet werden kann) umfassen, die mindestens für die Bilddatei, die eingelesenen Sensordaten und/oder die erste Position der anatomischen Struktur des Patienten als potentiell relevant eingestuft wird.

Das Verfahren kann ferner einen Schritt des Überprüfens des medizinischen Befunds hinsichtlich einer Konformität mit der medizinischen Richtlinie umfassen.

Das Verfahren kann ferner einen Schritt des Ausgebens eines Warnsignals umfassen, wenn der medizinische Befund nicht oder nicht vollständig konform mit der Richtlinie ist. Es ist auch möglich, dass der Grad der Abweichung codiert und gespeichert und abrufbar gemacht wird.

Die medizinische Richtlinie kann auch als "Standards of Operation (SOP)" bezeichnet werden.

Der medizinische Befund kann eine Abfolge von Diagnoseschritten umfassen, die vor und/oder nach der Aufnahme der Bilddatei erfolgt sein bzw. noch erfolgen können. Das Überprüfen des medizinischen Befunds hinsichtlich der Konformität mit der medizinischen Richtlinie kann eine Überprüfung umfassen, ob Diagnoseschritte gemäß der medizinischen Richtlinie laut medizinischem Befund eingehalten worden bzw. noch eingehalten werden. Alternativ oder ergänzend kann das Überprüfen des medizinischen Befunds hinsichtlich der Konformität mit der medizinischen Richtlinie eine Überprüfung umfassen, ob vorbestimmte Kombinationen von medizinischen Fachbegriffen in dem medizinischen Befund enthalten sind. Die medizinischen Fachbegriffe können sich auf eine oder mehrere Positionen einer anatomischen Struktur, insbesondere der ersten Position, beziehen, eine Diagnose und/oder eine Therapiemöglichkeit.

Bei einer Abweichung von der medizinischen Richtlinie, beispielsweise beim Fehlen einer Erstellung einer weiteren indizierten Differentialdiagnose und/oder eines Therapieplans, kann das Warnsignal ausgegeben werden.

Durch die Überprüfung des medizinischen Befunds hinsichtlich der Konformität mit der medizinischen Richtlinie kann die Qualität der Diagnose und Therapieplanung weiter verbessert werden.

Der Schritt des Überprüfens des medizinischen Befunds hinsichtlich einer Konformität mit der medizinischen Richtlinie kann ferner ein Identifizieren mindestens einer anatomischen Struktur in der medizinischen Richtlinie und ein Vergleichen der identifizierten anatomischen Struktur mit der anatomischen Struktur der mindestens einen ersten Position und/oder ein Vergleichen der identifizierten anatomischen Struktur mit der anatomischen Struktur der mindestens einen zweiten Position umfassen.

Der Schritt des Überprüfens des medizinischen Befunds hinsichtlich einer Konformität mit der medizinischen Richtlinie kann ferner ein Identifizieren mindestens einer vierten Position einer anatomischen Struktur in der medizinischen Richtlinie und ein Vergleichen der mindestens einen vierten Position mit der mindestens einen ersten Position und/oder ein Vergleichen der mindestens einen vierten Position mit der mindestens einen zweiten Position umfassen.

Durch die vorgenannten Schritte können in der Richtlinie solche anatomischen Strukturen automatisch erkannt werden, die bei der Befundung der Bilddatei gemäß der Richtlinie untersucht werden sollen. Durch den Abgleich mit der ersten bzw. zweiten Positionsinformation bzw. den damit verbundenen anatomischen Strukturen kann automatisch überprüft werden, ob a) die anatomischen Strukturen auf Grundlage der Sensordaten überhaupt in Augenschein genommen wurden und/oder b) ob die anatomischen Strukturen im Bericht behandelt wurden.

Der Schritt des Überprüfens des medizinischen Befunds hinsichtlich einer Konformität mit der medizinischen Richtlinie kann ferner eine Ausgabe einer Warnmeldung basierend auf dem Schritt des Vergleichens der identifizierten anatomischen Struktur mit der anatomischen Struktur der mindestens einen zweiten Position umfassen bzw. des Vergleichens der mindesten einen vierten Position mit der mindestens einen ersten Position und/oder der mindestens einen zweiten Position umfassen.

Dadurch kann automatisch eine Warnmeldung ausgegeben werden, wenn eine anatomische Struktur entgegen der medizinischen Richtlinie nicht befundet wurde.

Das Identifizieren der mindestens einen anatomischen Struktur des Patienten der mindestens einen vierten Position aus der medizinischen Richtlinie kann eine linguistische Datenverarbeitung eines Textes der medizinischen Richtlinie und eine anschließende Kodierung der aus dem linguistisch datenverarbeiteten Text extrahierten mindestens einen anatomischen Struktur umfassen. Optional kann die Kodierung Snomed und/oder RadLex umfassen.

Der Schritt des Überprüfens des medizinischen Befunds hinsichtlich einer Konformität mit der medizinischen Richtlinie kann einen durch Maschinelles Lernen und/oder künstliche Intelligenz (KI) trainierten Algorithmus umfassen.

Der Vergleich mit der medizinischen Richtlinie kann automatisiert und/oder auf einer zentralen Maschine, beispielsweise dem Server des KIS und/oder RIS, durchgeführt werden.

Vorstehend wurde die Lösung der Aufgabe anhand des Verfahrens beschriebenen. Dabei erwähnte Merkmale, Vorteile oder alternative Ausführungsformen sind ebenso auch auf die anderen beanspruchten Gegenstände (insbesondere die Vorrichtung, das System, das Computerprogrammprodukt und/oder das computerlesbare Speichermedium) zu übertragen und umgekehrt. Mit anderen Worten können auch die gegenständlichen Ansprüche (die beispielsweise auf eine Vorrichtung, ein System oder auf ein Computerprogrammprodukt gerichtet sind) mit den Merkmalen weitergebildet sein, die in Zusammenhang mit dem Verfahren beschrieben oder beansprucht sind und umgekehrt. Die entsprechenden funktionalen Merkmale des Verfahrens werden dabei durch entsprechende gegenständliche Module (beispielsweise jeweils als "-einheit" oder "-schnittstelle" bezeichnet), insbesondere durch Hardware-Module oder Mirkroprozessor-Module, der Vorrichtung, des Systems bzw. des Produktes ausgebildet und umgekehrt.

Gemäß einem Vorrichtungsaspekt wird eine Vorrichtung zur Sicherheitskontrolle eines medizinischen Befunds für einen Patienten basierend auf einer Bilddatei bereitgestellt, wobei die Bilddatei mittels eines bildgebenden medizinischen Geräts erfasst wurde. Die Vorrichtung umfasst eine Sensorschnittstelle, die zum Empfangen von Sensordaten mindestens eines Sensors bezüglich der Bilddatei ausgebildet ist. Die Vorrichtung umfasst ferner eine Zuordnungseinheit, die zum Zuordnen der eingelesenen Sensordaten zu einer ersten Position einer anatomischen Struktur des Patienten ausgebildet ist. Die Vorrichtung umfasst ferner eine Extraktionseinheit, die zum Extrahieren mindestens einer zweiten Position einer anatomischen Struktur des Patienten aus dem medizinischen Befund ausgebildet ist. Die Vorrichtung umfasst ferner eine Vergleichseinheit, die zum Vergleichen der ersten Position und der mindestens einen zweiten Position ausgebildet ist. Die Vorrichtung umfasst ferner eine Ausgabeschnittstelle, die zum Bereitstellen einer Ausgabe eines Warnsignals ausgebildet ist, falls der Vergleich keine Übereinstimmung ergibt. Gemäß einem Aspekt ist die Vorrichtung zur Ausführung des oben beschriebenen Verfahrens oder einer seiner Varianten ausgebildet.

Der mindestens eine Sensor kann einen Bewegungssensor, insbesondere einer Eingabeschnittstelle, und/oder einen Eingabesensor einer Eingabeschnittstelle umfassen, wobei die Eingabeschnittstelle eine Computermaus, ein Trackpad, einen Trackball, einen Joystick, eine Tastatur, und/oder einen berührungsempfindlicher Bildschirm umfassen kann. Alternativ oder ergänzend kann der mindestens eine Sensor einen Augen-Verfolgungssensor (auch: eye tracking sensor) umfassen. Alternativ oder ergänzend kann der mindestens eine Sensor einen Zeitmesser umfassen, der dazu ausgebildet ist, die Zeitdauer der Betrachtung einer Schicht aus einem Bildstapel oder einem Bereich in dem Bild, der in der Bilddatei codiert ist, zu messen.

Der Augen-Verfolgungssensor kann auch als "Eye Tracking Sensor" bezeichnet werden.

Die Vorrichtung kann ferner dazu ausgebildet sein, die Schritte des Verfahrens gemäß dem Verfahrensaspekt durchzuführen, und/oder Merkmale des Verfahrens gemäß dem Verfahrensaspekt zu umfassen.

Gemäß einem weiteren Aspekt wird ein System zur Sicherheitskontrolle eines medizinischen Befunds bereitgestellt. Das System umfasst mindestens einen Sensor zum Erfassen von Sensordaten bezüglich einer Bilddatei. Das System umfasst ferner eine Vorrichtung gemäß dem Vorrichtungsaspekt, wobei die Sensorschnittstelle der Vorrichtung die Sensordaten des mindestens einen Sensors einliest. Das System umfasst ferner optional eine Warnsignalausgabeeinrichtung, die basierend auf des an der Ausgabeschnittstelle der Vorrichtung bereitgestellten Warnsignals das Warnsignal ausgibt.

Gemäß einem weiteren Aspekt wird ein Computerprogrammprodukt bereitgestellt, umfassend Befehle, die bei der Ausführung des Programms durch einen Computer diesen veranlassen, das Verfahren gemäß dem Verfahrensaspekt auszuführen.

Gemäß einem weiteren Aspekt wird ein computerlesbares Speichermedium bereitgestellt, umfassend Befehle, die bei der Ausführung durch einen Computer diesen veranlassen, das Verfahren gemäß dem Verfahrensaspekt auszuführen.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: ein Ablaufdiagramm eines Verfahrens gemäß einer bevorzugten Ausführungsform der Erfindung;
- Fig. 2: ein Blockschaltbild einer Vorrichtung gemäß einer bevorzugten Ausführungsform der Erfindung; und
- Fig. 3: einen beispielhaften klinischen Arbeitsablauf, während dem die Schritte des erfindungsgemäßen Verfahrens ausführbar sind.

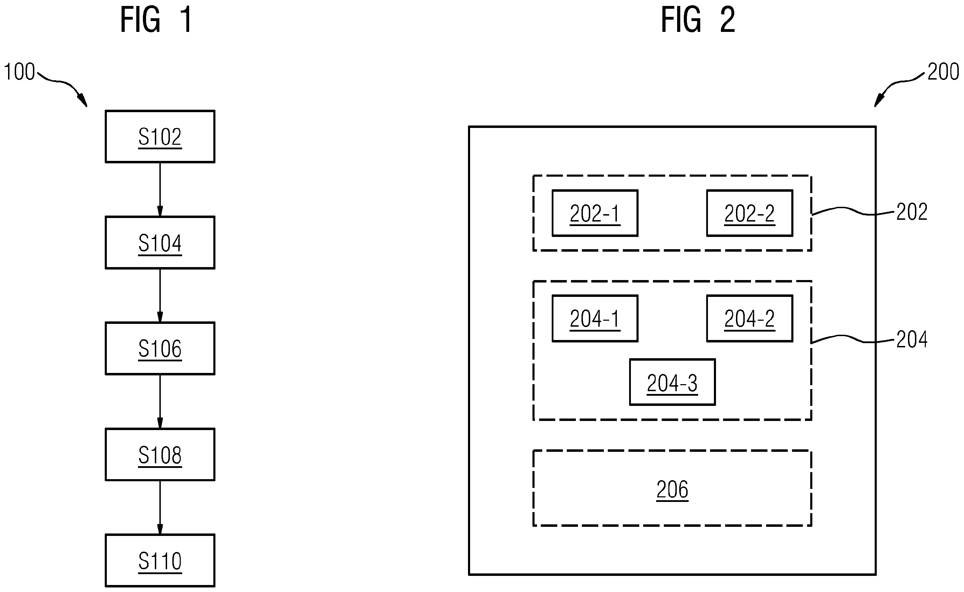

Fig. 1 zeigt beispielhaft ein computer-implementiertes Verfahren zur Sicherheitskontrolle eines medizinischen Befunds für einen Patienten basierend auf einer Bilddatei, wobei die Bilddatei mittels eines bildgebenden medizinischen Geräts erfasst wurde. Das Verfahren ist allgemein mit Bezugszeichen 100 bezeichnet.

In einem Schritt S102 werden Sensordaten bezüglich der Bilddatei eingelesen. In einem Schritt S104 werden die eingelesenen Sensordaten einer ersten Position einer anatomischen Struktur des Patienten zugeordnet. In einem Schritt S106 wird mindestens eine zweite Position einer anatomischen Struktur des Patienten aus dem medizinischen Befund extrahiert. In einem Schritt S108 wird die erste Position mit der mindestens einen zweiten Position verglichen. In einem Schritt S110 wird schließlich ein Warnsignal ausgegeben, falls der Vergleich S108 keine Übereinstimmung ergibt bzw. ergeben hat.

Fig. 2 zeigt beispielhaft eine Vorrichtung zur Sicherheitskontrolle eines medizinischen Befunds für einen Patienten basierend aus einer Bilddatei, wobei die Bilddatei mittels eines bildgebenden medizinischen Geräts erfasst wurde. Die Vorrichtung ist allgemein mit Bezugszeichen 200 bezeichnet.

Die in Fig. 2 gezeigte Vorrichtung 200 umfasst eine Sensorschnittstelle 202-1, die zum Empfangen von Sensordaten mindestens eines Sensors bezüglich der Bilddatei ausgebildet ist. Die in Fig. 2 gezeigte Vorrichtung 200 umfasst ferner eine Zuordnungseinheit 204-1, die zum Zuordnen der eingelesenen Sensordaten zu einer ersten Position einer anatomischen Struktur des Patienten ausgebildet ist. Die in Fig. 2 gezeigte Vorrichtung 200 umfasst ferner eine Extraktionseinheit 204-2, die zum Extrahieren mindestens einer zweiten Position einer anatomischen Struktur des Patienten aus dem medizinischen Befund ausgebildet ist. Die in Fig. 2 gezeigte Vorrichtung 200 umfasst ferner eine Vergleichseinheit 204-3, die zum Vergleichen der ersten Position und der mindestens einen zweiten Position ausgebildet ist. Die in Fig. 2 gezeigte Vorrichtung 200 umfasst ferner eine Ausgabeschnittstelle 202-2, die zum Bereitstellen einer Ausgabe eines Warnsignals ausgebildet ist, falls der Vergleich keine Übereinstimmung ergibt.

Gemäß einem Ausführungsbeispiel, das mit jedem anderen Ausführungsbeispiel kombinierbar ist, können die Sensorschnittstelle 202-1 und die Ausgabeschnittstelle 202-2 in einer Datenschnittstelle 202 der Vorrichtung 200 zusammengefasst sein.

Gemäß einem weiteren Ausführungsbeispiel, das mit jedem anderen Ausführungsbeispiel kombinierbar ist, können die Zuordnungseinheit 204-1, die Extraktionseinheit 204-2 und die Vergleichseinheit 204-3 in einer (insbesondere gemeinsamen) Recheneinheit und/oder und einem Prozessor 204 angeordnet sein.

Gemäß einem weiteren Ausführungsbeispiel, das mit jedem anderen Ausführungsbeispiel kombinierbar ist, kann die Vorrichtung 200 ferner einen Datenspeicher 206 umfassen.

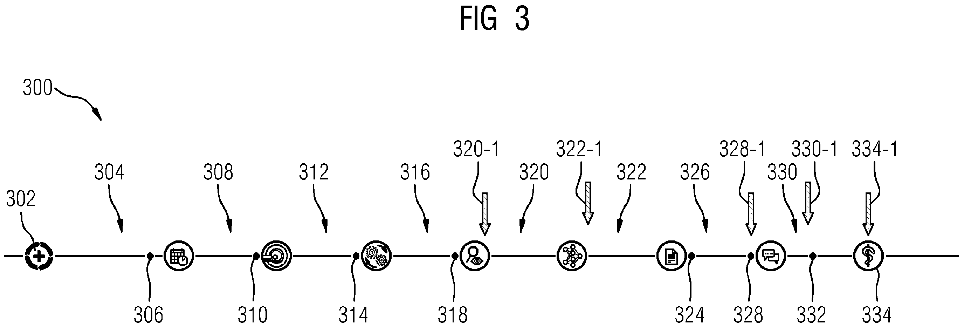

Fig. 3 zeigt ein Beispiel eines Arbeitsflusses 300, während dem das erfindungsgemäße Verfahren ausgeführt wird.

Während der herkömmlichen Befundung radiologischer Bilddateien (kurz: Bilder) fokussiert das geschulte medizinische Personal (auch: der Arzt bzw. die Ärztin und/oder die/der Nutzer:in der Bildbetrachtungssoftware), welches eine Bildbetrachtungssoftware benutzt, auf Bildbereiche, die auffällig sind, die auf eine Pathologie hinweisen, und/oder welche durch eine medizinische Fragestellung eine besondere Aufmerksamkeit erfordern.

Erfindungsgemäß können folgende Sensordaten (die auch als Informationen bezeichnet werden können) genutzt werden, um zu verstehen auf welche Bildbereiche sich das geschulte medizinische Personal (auch: der Arzt bzw. die Ärztin und/oder die/der Nutzer:in der Bildbetrachtungssoftware) während des Lesens des Befundes fokussiert hat. Bildbereiche, auf die sich das geschulte medizinische Personal beim Betrachten der Bilddatei und/oder beim Lesen des Befunds fokussiert hatten, können auch als Fokusbereiche bezeichnet werden.

Gemäß einem ersten Ausführungsbeispiel, das mit jedem anderen Ausführungsbeispiel kombinierbar ist, wird (z.B. als Sensordaten) eine Hitzekarte (englisch/fachsprachlich auch: Heatmap) erzeugt aus den länger betrachteten Schichten (englisch/fachsprachlich auch: Slices) in einem Bildstapel. Aus der Hitzekarte können ein oder mehrere Fokusbereiche bestimmt werden.

Alternativ oder ergänzend können (z.B. als Sensordaten) Bewegungsdaten einer Eingabeeinrichtung und/oder einer Blickerfassung (beispielhaft englisch/fachsprachlich auch: Mouse-Tracking und/oder Eye-Tracking) verwendet werden, um Fokusbereiche (auch: fokussierte Bildbereiche) auf einer Schicht (auch: Schnittbildebene) oder auf einem zweidimensionalen Bild zu erkennen.

Gemäß einem zweiten Ausführungsbeispiel, das mit jedem anderen Ausführungsbeispiel kombinierbar ist, werden (z.B. als Sensordaten) händische Messungen und/oder Annotationen, insbesondere ein "Bereich von Interesse" (englisch: region of interest; kurz: ROI) oder Distanzmessung zur Bestimmung eines Fokusbereichs verwendet. Alternativ oder ergänzend können Messwerte der Messungen, z.B. ein Durchmesser und/oder Dichte (beispielsweise einer anatomischen Struktur, insbesondere eines Organs oder einer Läsion), zur Bestimmung des Fokusbereichs verwendet werden.

Gemäß einem dritten Ausführungsbeispiel, das mit jedem anderen Ausführungsbeispiel kombinierbar ist, können ein oder mehrere Fokusbereiche aufgrund einer Ausführung eines spezifischen Hilfsprogramms (auch: Tools) und/oder sonstiger Bedienungsmuster bestimmt werden (z.B. jeweils als Sensordaten). Das spezifische Hilfsprogramm kann ein Hilfsprogramm der Software "Medical Imaging Interaction Toolkit" (MITK) umfassen und/oder beispielsweise eine Segmentierung und/oder Annotationen.

Um diese Datenpunkte einer (z.B. ersten) Position einer anatomischen Struktur (kurz: anatomischen Position; auch: anatomischen Lokation) zuzuordnen, kann ein Algorithmus die auf die Bilddatei (oder das Bild) bezogenen Bereiche und/oder Punkte den (z.B. ersten) anatomischen Positionen (und/oder anatomischen Lokationen) zuordnen. Dies kann beispielsweise über (beispielsweise ALPHA-) Landmarken, (insbesondere Organ-) Segmentierungen und/oder einen explizit für diesen Zweck angelernten Machine Learning (ML) Algorithmus (beispielsweise mit Deep Learning, DL) erfolgen. Das Ausgabeformat der (z.B. ersten) Position der anatomischen Struktur (und/oder anatomischen Lokation) erfolgt dabei vorteilhafterweise in einer gängigen Kodierung (beispielsweise.: Snomed und/oder RadLex). Werden die fokussierten Bildbereiche (und/oder Sensordaten) während der Befundung erfasst und einer oder mehreren (z.B. ersten) Positionen anatomischer Strukturen (und/oder anatomischen Lokationen) zugeordnet, so kann überprüft werden, ob sich zu jedem fokussierten Bildbereich (und/oder allen Sensordaten) eine Übereinstimmung (auch bezeichnet als semantisches Korrelat) im Befund befindet.

Die Überprüfung einer Übereinstimmung (und/oder eines semantischen Korrelats) kann abhängig von der Befundart erfolgen. Im Weiteren wird zwischen weitestgehend unstrukturierten Befunden und strukturierten Befunden unterschieden.

Für unstrukturierte Befunde (insbesondere weitestgehend Fließtext, beispielsweise diktiert) wird in einem ersten Schritt mit Hilfe von Natural Language Processing erkannt, welche (z.B. mindestens eine zweite) Positionen anatomischer Strukturen (und/oder welche anatomischen Lokationen) bereits im Befund erwähnt werden.

In einem zweiten Schritt werden die aus dem Befund extrahierten (z.B. mindestens eine zweiten) Positionen anatomischer Strukturen (und/oder extrahierten anatomischen Lokation) in eine, insbesondere gängige, Kodierung (beispielsweise.: Snomed und/oder RadLex) übersetzt.

In einem dritten Schritt werden die kodierten (z.B. mindestens einen zweiten) Positionen anatomischer Strukturen (und/oder anatomischen Lokationen) aus dem Befundtext mit der einen oder den mehreren (z.B. ersten) Positionen anatomischer Strukturen (und/oder anatomischen Lokationen) aus den Sensordaten (und/oder den fokussierten Bildbereichen) verglichen.

Für strukturierte Befunde (insbesondere umfassend einen geordneten Text und eine strukturierte kodierte Metainformationen) liegen die (z.B. mindestens eine zweiten) Positionen anatomischer Strukturen (und/oder anatomischen Lokationen) im strukturierten Befund bereits in kodierter Form (z.B. nach Standard) vor.

In einem Schritt werden die kodierten (z.B. mindestens eine zweiten) Positionen anatomischer Strukturen (und/oder die kodierten anatomischen Lokationen) aus dem strukturierten Befund mit der einen oder den mehreren (z.B. ersten) Positionen anatomischer Strukturen (und/oder anatomischen Lokationen) aus den Sensordaten (und/oder aus den fokussierten Bildbereichen) verglichen.

Falls im aktuellen Befund Übereinstimmungen und/oder Korrelate zu fokussierten Bildbereichen und/oder einem oder mehreren (z.B. ersten) Positionen anatomischer Strukturen, die Sensordaten zugeordnet sind, fehlen, so kann eine Nutzerin oder ein Nutzer, insbesondere geschultes medizinisches Personal und/oder ein Arzt oder Ärztin, auf unterschiedliche Art und Weisen darauf hingewiesen werden.

Gemäß einem Ausführungsbeispiel, das mit jedem anderen Ausführungsbeispiel kombinierbar ist, werden beim Versuch, den Befund abzuschließen (und/oder beim Signing) alle ersten Positionen anatomischer Strukturen (und/oder fokussierten Bildbereiche) aufgezählt und visualisiert, für welche es keine Übereinstimmung und/oder kein semantisches Korrelat im Befund gibt. Erst wenn die Autorin oder der Autor des Befunds (und/oder die Unterzeichnerin oder der Unterzeichner und/oder der/die Nutzer:in) bestätigt, dass die fehlende Übereinstimmung (und/oder das fehlende semantische Korrelat) beabsichtigt war, wird der Befund finalisiert.

Gemäß einem weiteren Ausführungsbeispiel, das mit jedem anderen Ausführungsbeispiel kombinierbar ist, wird während des Befundens der Autorin oder dem Autor (und/oder dem/der Nutzer:in) eine schematische Ansicht des betrachteten Körperbereichs angezeigt, in welchem erste Positionen anatomischer Strukturen (und/oder fokussierte Bildbereiche) durch eine Signalfarbe (z.B. rot) markiert werden, die nicht im Befund erwähnt sind. Erste Positionen anatomischer Strukturen (und/oder fokussierte Bildbereiche), die bereits im Befund Erwähnung gefunden haben, werden entweder nicht markiert oder durch eine Erfolgsfarbe (z.B. grün) markiert. Vorteilhafterweise unterscheidet sich die Erfolgsfarbe von der Signalfarbe.

Gemäß einem weiteren Ausführungsbeispiel, das mit jedem anderen Ausführungsbeispiel kombinierbar ist, wird während des Befundens der Autorin oder dem Autor (und/oder dem/der Nutzer:in) im Befund, beispielsweise seitlich, ein Warnzeichen angezeigt, welches beim Übergleiten (und/oder Hovern) mit der Computermaus über erste Positionen anatomischer Strukturen (und/oder fokussierte Bildbereiche) informiert (und/oder ein entsprechendes Warnsignal ausgibt), die keine Übereinstimmung und/oder kein semantisches Korrelat im Befundtext haben. Über einen Klick kann die Autorin oder der Autor (und/oder der/die Nutzer:in) zu der ersten Position und/oder dem fokussierten Bildbereich springen. In einem strukturierten Befund können diese Warnsignale (auch: Warnzeichen) gezielt an jenen Stellen im Befund und/oder im Befundtemplate platziert werden, wo ein entsprechendes semantisches Korrelat erwartet werden würde.

Alternativ oder ergänzend kann die Technik (insbesondere das im System ausgeführte Verfahren) auch warnen, wenn ein Ergebnis einer künstlichen Intelligenz (auch: KI Resultat und/oder AI Resultat) von der Autorin oder dem Autor (und/oder dem/der Nutzer:in) weder akzeptiert noch abgelehnt wurde.

Die erfindungsgemäße Technik (insbesondere das beschriebene Verfahren, die Vorrichtung und/oder das System) unterstützt befundendes geschultes medizinisches Personal, insbesondere Radiologinnen und/oder Radiologen, bei der Erstellung eines vollständigen und konsistenten Befunds. Die erfindungsgemäße Technik führt eine weitere (beispielsweise zusätzliche zu herkömmlichen) Sicherheitsebene ein, die es ermöglicht, die Qualität der Befunde zu steigern und Fehler in der Befundung weiter zu minimieren.

Die Steigerung der Qualität der Befunde und die Minimierung von Fehlern in der Befundung hat Vorteile für die Patienten, da eventuelle aus einer Fehlbefundung resultierende Behandlungsfehler minimiert werden können. Darüber hinaus beschleunigt die erfindungsgemäße Technik den Überprüfungsprozess (und/oder den Supervidierungsprozess), da die supervidierende Oberärztin oder der supervidierende Oberarzt seltener nachkorrigieren muss.

Medizinische Richtlinien und/oder Standardarbeitsanweisungen (SOP) enthalten Empfehlungen und/oder Regeln dafür, wie bestimmte medizinische Daten eines Patienten analysiert werden sollten, um eine bestimmte Diagnose zu stellen oder auszuschließen. Medizinische Richtlinien und/oder SOPs können angeben, welche Diagnoseschritte und/oder Messungen durchgeführt werden sollten, und/oder welche Datenverarbeitungswerkzeuge auf die verfügbaren Daten (insbesondere die Daten der Bilddatei und/oder die Sensordaten) anzuwenden sind, wenn ein bestimmter medizinischer Zustand eines Patienten diagnostiziert wird. Außerdem kann in medizinischen Richtlinien festgelegt sein, welche Folgemaßnahmen in Abhängigkeit von den Ergebnissen der diagnostischen Schritte durchgeführt werden sollten. Einige medizinische Richtlinien enthalten Informationen über die Vorbeugung und die Prognose bestimmter Erkrankungen sowie über die Risiken und/oder den Nutzen. Richtlinien, die von Regierungen, medizinischen und berufsständischen Gruppen und anderen Stellen entwickelt werden, sollen unnötige Tests und Untersuchungen sowie Überbehandlungen und/oder Unterbehandlungen vermeiden. Werden die Richtlinien nicht befolgt, kann dies zu suboptimalen und/oder schädlichen diagnostischen und/oder therapeutischen Folgemaßnahmen führen.

Die in einer Richtlinie enthaltenen Informationen sind in der Regel spezifisch für ein bestimmtes medizinisches Fachgebiet und/oder für einen bestimmten Gesundheitszustand und/oder die verfügbaren medizinischen Daten eines Patienten. Infolgedessen sind klinische Richtlinien herkömmlicherweise sehr komplex. So umfasst beispielsweise das geltende Behandlungsablaufdiagramm des US-amerikanischen National Comprehensive Cancer Network (NCCN) fast 200 Seiten. Für Ärztinnen und Ärzte ist es daher herkömmlicherweise schwierig, diese Richtlinien bei der Lektüre eines Patientenfalls zu beachten, da es gar nicht so einfach ist, die Richtlinien in ihrer Gesamtheit auswendig zu lernen und anzuwenden. Insbesondere wenn eine Radiologin oder ein Radiologe an einer Lese- und Befundstation (beispielsweise einem Computer und/oder einer Vorrichtung 200) mit einem nicht standardisierten Fall konfrontiert wird, ist es herkömmlicherweise sehr schwierig, die Richtlinien korrekt anzuwenden. Außerdem kann ein Nutzer (beispielsweise geschultes medizinisches Personal, insbesondere eine Radiologin oder ein Radiologe) herkömmlicherweise in der klinischen Routine versucht sein, bestimmte Prüfpunkte einer Richtlinie zu überspringen, um Zeit zu sparen - nicht wissend, was das Auslassen eines bestimmten Schritts für den weiteren klinischen Weg bedeuten könnte.

Herkömmlicherweise wird dieses Problem gelöst, indem der Nutzer vor oder während der Lektüre eines Patientenfalls auf die entsprechende Richtlinie aufmerksam gemacht wird. Ein Problem bei diesem herkömmlichen Ansatz ist, dass der Nutzer in der täglichen Routine oft nicht die Zeit hat, sich mit den Richtlinien vertraut zu machen, um festzustellen, welche Schritte anzuwenden sind. Darüber hinaus können sich insbesondere fachkundige Nutzer durch solche Maßnahmen bevormundet fühlen, da sie in der Regel (z.B. in Standardfällen) wissen, was sie zu tun haben, um Richtlinienkonform zu handeln bzw. wo geringfügige Abweichungen möglich sind, ohne Probleme für die nachgelagerte Diagnose oder Behandlungsentscheidungen für den Patienten zu verursachen.

Die hier vorgeschlagene Technik erkennt gemäß einem Ausführungsbeispiel das mit jedem anderen Ausführungsbeispiel kombinierbar ist, automatisch, ob und wie ein Nutzer (beispielsweise geschultes medizinisches Personal, insbesondere eine Radiologin oder ein Radiologe) beim Lesen eines Patientenfalls und insbesondere medizinischer Bilddaten (beispielsweise, umfassend die Bilddatei und/oder die Sensordaten) von der geltenden medizinischen Richtlinie abweicht. Zu diesem Zweck können die vom Nutzer (beispielsweise dem geschulten medizinischen Personal, insbesondere der Radiologin oder dem Radiologe) durchgeführten Aktionen und die von ihm gezogenen Schlussfolgerungen automatisch auf ihre Übereinstimmung mit der geltenden medizinischen Richtlinie analysiert werden.

Das Verfahren kann einen Schritt des Zugreifens auf das Patientendatenmodell und Auslesen der verfügbaren Patientendaten (beispielsweise Bilddaten - insbesondere umfassend die Bilddatei und die zugehörigen Sensordaten - , Nicht-Bilddaten, frühere Berichte, Arbeitslisteneintrag für den Nutzer, und/oder Aufgabe für den Nutzer) umfassen.

Das Verfahren kann ferner einen Schritt des Identifizierens der anwendbaren medizinischen Richtlinie und/oder SOP umfassen auf der Grundlage der verfügbaren Patientendaten.

Das Verfahren kann ferner optional einen Schritt des Bestimmens des Stadiums (beispielsweise hinsichtlich eines Gesundheitszustands und/oder einer Behandlung) des Patienten in der identifizierten Richtlinie umfassen.

Das Verfahren kann ferner einen Schritt des Überwachens der Nutzeraktionen durch direkte Überwachung der Nutzereingaben in der Lese- und Befundstation (beispielsweise dem Computer und/oder der Vorrichtung 200) und/oder durch Analyse des medizinischen Berichts des Nutzers umfassen, sobald dieser abgezeichnet (auch: signiert) wurde.

Es kann überprüft werden, welche Hilfsprogramme (auch: Tools) verwendet wurden. Alternativ oder ergänzend kann überprüft werden, ob es künstliche Intelligenz (KI)-basierte Ergebnisse KI-basierter Hilfsmittel (auch: KI-basierter Tools) gibt. Falls es KI-basierte Ergebnisse gibt, kann mindestens ein Vertrauenswert (auch: Konfidenzwert) überprüft werden. Weiterhin alternativ oder ergänzend kann überprüft werden, welche Elemente der verfügbaren Patientendaten geöffnet und/oder eingesehen wurden.

Weiterhin alternativ oder ergänzend kann überprüft werden, welche Messungen durchgeführt wurden. Weiterhin alternativ oder ergänzend kann überprüft werden, welche Messungen im Bericht berücksichtigt wurden. Weiterhin alternativ oder ergänzend kann überprüft werden, welche Diagnose formuliert wurde. Weiterhin alternativ oder ergänzend kann überprüft werden, welche Differentialdiagnose oder Differentialdiagnosen in Betracht gezogen wurden. Weiterhin alternativ oder ergänzend kann überprüft werden, welche Behandlungsoptionen empfohlen werden. Weiterhin alternativ oder ergänzend kann überprüft werden, welche Nachuntersuchungen berücksichtigt wurden oder in Zukunft berücksichtigt werden sollen.

Das Verfahren kann ferner einen Schritt des Vergleichens der überwachten Nutzerhandlung (beispielsweise die Diagnose und/oder Therapieschritte) mit der ermittelten Richtlinie unter Berücksichtigung des ermittelten Stadiums umfassen.

Das Verfahren kann ferner einen Schritt des Ermittelns einer Abweichung von der Richtlinie umfassen. Eine Abweichung kann umfassen, dass ein (beispielsweise KI-) Hilfsmittel (auch: Tool) nicht auf die Patientendaten angewendet wurde.

Alternativ oder ergänzend kann eine Abweichung umfassen, dass eine Messung nicht durchgeführt wurde.

Alternativ oder ergänzend kann eine Abweichung umfassen, dass Aspekte der Patientendaten nicht berücksichtigt wurden, z. B. Nicht-Bilddaten wie Labordaten, bestimmte Abschnitte einer Bildstudie und/oder eine verfügbare frühere Studie.

Alternativ oder ergänzend kann eine Abweichung bedeuten, dass keine sinnvollen Bewertungen (englisch: Scores), z. B. BI-RADS-Merkmale (BI-RADS kurz für: Breast Imaging-Reporting and Data System), aus einem Bericht extrahiert werden können.

Alternativ oder ergänzend kann eine Abweichung umfassen, dass zusätzliche, nicht in der SOP vorgesehene Tests durchgeführt wurden und/oder geplant sind. Alternativ oder ergänzend kann eine Abweichung umfassen, dass nicht optimale Tests (z. B. MRT statt Ultraschall als Bildgebungsmodus) durchgeführt wurden und/oder geplant sind.

Das Verfahren kann ferner optional einen Schritt des Bestimmens einer Kritikalität der Abweichung von der Richtlinie umfassen. Dabei wird die Kritikalität der Abweichung vorzugsweise quantifiziert und/oder in ein ausgebbares Format gewandelt. Einige Auslassungen und/oder Abweichungen von der Richtlinie, beispielsweise von Nebendiagnosen, haben möglicherweise nur begrenzte Auswirkungen auf die Patientenverfolgung (auch: Patientenreise oder Patientenwerdegang), während andere Auslassungen und/oder Abweichungen von der Richtlinie den Patienten auf einen völlig anderen (insbesondere Therapie-) Weg bringen können. Die Kritikalität kann z. B. durch eine Anzahl der Knotenpunkte abgeschätzt werden, die bei einer fehlerhaften Anwendung der Richtlinien anders verlaufen würden als bei einem wahrscheinlichen Verlauf.

Das Verfahren kann ferner, abhängig von der Kritikalität, einen Schritt des Informierens des Nutzers umfassen, dass eine Abweichung von der Richtlinie (insbesondere mit einer bestimmten Kritikalität) vorliegt, und/oder welche anatomischen Bereiche oder was zusätzlich zu den Nutzeraktionen überprüft werden muss, um die Abweichung von der Richtlinie rückgängig zu machen. Je nach Kritikalität (beispielsweise bei einer Kritikalität über einem vorbestimmten Schwellwert) kann ein zuvor vom Nutzer abgezeichneter medizinischer Bericht zurückgewiesen werden, bis die Abweichung behoben ist.

Das Verfahren kann ferner einen Schritt des Bestimmens der Konsequenzen der Abweichung von der Richtlinie für die weitere Patientenverfolgung umfassen und optional den Nutzer auf die Konsequenzen aufmerksam machen, um das Verständnis des Nutzers dafür zu fördern, dass bestimmte kritische Abweichungen von der Richtlinie im Sinne eines erklärbaren (insbesondere KI-) Hilfsmittels (auch: Tools) behoben werden müssen. Solche Konsequenzen könnten beispielweise die Notwendigkeit einer erneuten Untersuchung des Patienten umfassen und/oder eine Behandlungsempfehlung aufgrund von Befunden mit geringerem Vertrauenswert (auch: geringer Konfidenz).

Das Verfahren kann ferner umfassen, dass für den Fall, dass die Abweichung als sinnvoll erachtet wird, z.B. aufgrund nicht erfasster Informationen in der elektronischen Patientenakte, die notwendigen Dokumentationen in der Patientenakte hinsichtlich hinzugefügter oder übersprungener Schritte automatisch zusammen mit einer Begründung des geschulten medizinischen Personals, insbesondere eines verantwortlichen Arztes, ausgeführt werden.

Fig. 3 zeigt beispielhaft Stadien eines klinischen Arbeitsablaufs 300, in denen das erfindungsgemäße Verfahren 100 angewendet werden kann.

Der klinische Arbeitsablauf (auch "Workflow") 300 beginnt an Bezugszeichen 302 mit einem oder mehreren Kontrollpunkten. An Bezugszeichen 304 wird eine medizinische Indikation festgestellt. An Bezugszeichen 306 wird die Entscheidung getroffen, mindestens eine Bilddatei mittels einer bildgebenden Modalität, beispielsweise mittels MRT und/oder CT, aufzunehmen, bzw. die Aufnahme durch das medizinische Gerät initiiert. An Bezugszeichen 308 wird eine Terminierung (auch: Scheduling) bestimmt und an Bezugszeichen 310 ein Protokoll zur Bildaufnahme (auch: Scan-Protokoll) ausgewählt. An Bezugszeichen 312 wird die mindestens eine Bilddatei entsprechend dem ausgewählten Protokoll aufgenommen. An Bezugszeichen 314 wird eine Verarbeitungsart (auch: Processing) ausgewählt und an Bezugszeichen 316 auf die mindestens eine aufgenommene Bilddatei angewendet. An Bezugszeichen 318 wird eine Reihenfolge des Lesens der Bilddatei ausgewählt, beispielsweise wenn die Bilddatei einen Bildstapel von Schichten oder 3- oder mehrdimensionale Daten umfasst und/oder eine zeitliche Abfolge von Bildern.

An Bezugszeichen 320 wird die mindestens eine Bilddatei von geschultem medizinischem Personal, insbesondere einem Radiologen, gelesen und bei Bezugszeichen 322 Schlussfolgerungen getroffen.

Verfahrensschritte können an Bezugszeichen 320-1 während der Lesephase 320 und/oder an Bezugszeichen 322-1 während der Phase 322 der Schlussfolgerungen ausgeführt werden (beispielsweise an einem Leseplatz, insbesondere einem Computer oder einer Vorrichtung 200). Insbesondere kann das geschulte medizinische Personal, insbesondere ein Facharzt (z.B. ein Radiologe, und/oder Pathologe) eine Richtlinienprüfung initiieren, z.B. durch Anklicken einer Aktionsschaltfläche. Die Richtlinienprüfung während der Lesephase 320 und/oder der Schlussfolgerungsphase 322 kann auch als "online" bezeichnet werden.

An Bezugszeichen 324 wird eine Befundart und/oder eine medizinische Diagnose ausgewählt und über eine Phase 326 der Befund erstellt. Der Befund wird an Bezugszeichen 328 (beispielsweise durch den Autor und/oder Ersteller des Befunds) abgezeichnet.

Sobald der medizinische Bericht, insbesondere von einem Facharzt (z.B. am lesenden Arbeitsplatz) abgezeichnet wurde, kann an Bezugszeichen 328-1 eine automatische Richtlinienüberprüfung (und/oder eine Überprüfung der Einhaltung der Richtlinien) initiiert werden. Alternativ oder ergänzend kann an Bezugszeichen 328-1 die Richtlinienprüfung automatisch ausgelöst werden, wenn ein Auftrag (beispielsweise der Befund) abgeschlossen ist und/oder ein Bearbeitungsprogramm des Befunds geschlossen wird.

Bezugszeichen 330 zeigt eine Phase einer zentralen Qualitätskontrolle für medizinische Berichte einer Einrichtung. An Bezugszeichen 332 wird ein Ergebnis und/oder Bericht der Qualitätskontrolle ausgegeben.

Eine Überprüfung 330-1 der Einhaltung der Richtlinien kann während der Qualitätskontrollphase 330 erfolgen. Die Überprüfung außerhalb oder unabhängig von dem Lese- und Berichtsarbeitsablauf 320 bzw. 322 kann auch als "offline" bezeichnet werden. Eine zentrale Entscheidungsmaschine kann Teil eines Krankenhaus-Informations-Systems (KIS) und/oder eines Radiologie-Informations-Systems (RIS) oder Ähnlichem sein.

Falls eine Begründung für Abweichungen von den Richtlinien im Bericht fehlt, kann die Begründung nachträglich, insbesondere während der Qualitätskontrollphase 330 angefordert werden, um die Dokumentation der Patientenverfolgung zu vervollständigen.

Eine Überprüfung 334-1 der Einhaltung der Richtlinien kann an einem bestimmten Entscheidungspunkt in der klinischen Richtlinie initiiert werden, z. B. wenn eine Therapieentscheidung (auch: Behandlungsentscheidung) vom behandelnden Arzt (wie dem Chirurgen, Strahlentherapeuten und/oder einem anderen behandelnden Arzt und/oder von einem Ärzteteam in einem Tumorboard) getroffen wird, beispielsweise an Bezugszeichen 334. Die Richtlinienüberprüfung 334-1 kann automatisch auf der Grundlage eines Kontrollpunktes im Arbeitsablauf, beispielsweise an Bezugszeichen 334, und/oder durch geschultes medizinisches Personal, insbesondere den behandelnden Arzt, erfolgen. Das geschulte medizinische Personal, insbesondere der behandelnde Arzt kann eine Vielzahl von Einzeldiagnosen und/oder Berichten aggregieren und bewerten, um zu einer Behandlungsentscheidung zu gelangen. Dabei kann mehr als ein medizinischer Bericht, beispielsweise alle für den Patienten relevanten Berichte (z.B. Radiologieberichte, Laborberichte, und/oder Pathologieberichte) auf die Einhaltung der Richtlinie, und insbesondere auf das Vorhandensein aller relevanten Werte, überprüft werden. Auf diese Weise kann der behandelnde Arzt beurteilen, ob möglicherweise Informationen fehlen oder nicht.

Die Übereinstimmung eines Berichts mit einer Richtlinie kann beispielsweise bewertet werden, indem ein (insbesondere KI-) Modell trainiert wird. Das (insbesondere KI-) Modell kann trainiert werden, indem akzeptable (beispielsweise von einer Qualitätskontrolle als den Qualitätsstandards genügend) Berichte mit den entsprechenden Anforderungen aus der entsprechenden Richtlinie und/oder SOP verglichen werden.

Durch eine (insbesondere automatisierte) Richtlinienüberprüfung kann eine verbesserte Qualität der Ergebnisse und/oder Schlussfolgerungen aus der Bilddatei (und/oder weiteren Untersuchungen und/oder Befunden) ermöglicht werden. Alternativ oder ergänzend kann durch die (insbesondere automatisierte) Richtlinienüberprüfung eine Absicherung und/oder Dokumentation der Richtlinienkonformität ermöglicht werden. Weiterhin alternativ oder ergänzend kann durch die (insbesondere automatisierte) Richtlinienüberprüfung eine Transparenz (beispielsweise der Diagnosestellung und/oder Behandlungsentscheidung) erhöht werden. Weiterhin alternativ oder ergänzend können erklärbare Ergebnisse die Akzeptanz der (insbesondere automatischen) Überprüfungen, beispielsweise der Richtlinienüberprüfungen, fördern.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für Bilddateien eines einzelnen bildgebenden medizinischen Geräs angewendet werden kann, sondern auch für eine Kombination bildgebender medizinischer Geräte sowie eine Kombination anderweitiger medizinischer Diagnosetechniken. Des Weiteren können die Bauteile der Vorrichtung und/oder des Systems auf mehreren physikalischen Produkten verteilt realisiert werden.

## Patentansprüche

1. Computer-implementiertes Verfahren zur Sicherheitskontrolle eines medizinischen Befunds für einen Patienten basierend auf einer Bilddatei, die mittels eines bildgebenden medizinischen Geräts erfasst wurde, umfassend die Schritte:
- Einlesen (S102) von Sensordaten bezüglich der Bilddatei;
- Zuordnen (S104) der eingelesenen Sensordaten zu einer ersten Position einer anatomischen Struktur des Patienten;
- Extrahieren (S106) mindestens einer zweiten Position einer anatomischen Struktur des Patienten aus dem medizinischen Befund;
- Vergleichen (S108) der ersten Position und der mindestens einen zweiten Position; und
Ausgeben (S110) eines Warnsignals, falls der Vergleich (S108) keine Übereinstimmung ergibt.

2. Verfahren nach Anspruch 1, wobei die Sensordaten ausgewählt sind aus der Gruppe bestehend aus:
- einer Zeitdauer einer Darstellung, insbesondere auf einem Bildschirm, einer Bildschicht innerhalb eines Bildstapels medizinischer Bilder der Bilddatei;
- Bewegungsdaten einer Eingabeeinrichtung, insbesondere einer Computermaus, auf einer Darstellung eines Bildes oder einer Bildschicht der Bilddatei;
- Bewegungsdaten einer Blickerfassung auf einer Darstellung eines Bildes oder einer Bildschicht der Bilddatei;
- Markierungen eines Bereichs von Interesse auf einer Darstellung eines Bildes oder einer Bildschicht der Bilddatei;
- Messungen einer Distanz, eines Durchmessers und/oder einer Dichte auf einer Darstellung eines Bildes oder einer Bildschicht der Bilddatei;
- Annotationen zu einer Darstellung eines Bildes oder einer Bildschicht der Bilddatei; und
Bedienungsdaten hinsichtlich einer Darstellung mindestens eines Bildes der Bilddatei.

3. Verfahren nach Anspruch 1 oder 2, wobei das Zuordnen (S104) der eingelesenen (S102) Sensordaten zu der ersten Position der anatomischen Struktur des Patienten ein Identifizieren von anatomischen Landmarken, eine Segmentierung, insbesondere mindestens eines Organs des Patienten.

4. Verfahren nach einem der Ansprüche 1 bis 3, wobei das Zuordnen (S104) der eingelesenen (S102) Sensordaten zu der ersten Position der anatomischen Struktur des Patienten einen Schritt des Ausgebens des Ergebnisses des Zuordnens (S104) in einer Kodierung umfasst, wobei die Kodierung optional Snomed und/oder RadLex umfasst.

5. Verfahren nach einem der Ansprüche 1 bis 4, wobei das Extrahieren (S106) der mindestens einen zweiten Position der anatomischen Struktur des Patienten aus dem medizinischen Befund eine linguistische Datenverarbeitung eines Textes des medizinischen Befunds und eine anschließende Kodierung der aus dem linguistisch datenverarbeiteten Text extrahierten mindestens einen zweiten Position umfasst.

6. Verfahren nach einem der Ansprüche 1 bis 5, wobei das Extrahieren (S106) der mindestens einen zweiten Position der anatomischen Struktur des Patienten aus dem medizinischen Befund ein Ausgeben in einer Kodierung umfasst.

7. Verfahren nach Anspruch 4 in Kombination mit Anspruch 5 oder 6, wobei das Vergleichen der den eingelesenen (S102) Sensordaten zugeordneten ersten Position mit der mindestens einen aus dem medizinischen Befund extrahierten (S106) zweiten Position einen Vergleich der jeweiligen Kodierungen umfasst.

8. Verfahren nach einem der Ansprüche 1 bis 7, wobei das Warnsignal ausgewählt ist aus der Gruppe bestehend aus:
- einer Ausgabe der den eingelesenen (S102) Sensordaten zugeordneten ersten Position, für die im Schritt des Vergleichens (S108) keine entsprechende aus dem medizinischen Befund extrahierte (S106) zweiten Position gefunden wurde, auf einer Darstellung eines Bildes oder einer Schicht einer 3D-Bilddatei;
- einer Textausgabe einer fehlenden Übereinstimmung hinsichtlich der den eingelesenen Sensordaten (S102) zugeordneten ersten Position; und
einer Blockierung eines Arbeitsschritts des Abschließens und/oder Unterzeichnens des medizinischen Befunds.

9. Verfahren nach einem der Ansprüche 1 bis 8, wobei das medizinische bildgebende Gerät ausgewählt ist aus der Gruppe bestehend aus:
- einem Röntgengerät;
- einem Magnetresonanztomograph, MRT;
- einem Positronen-Emissions-Tomograph, PET;
- einem Angiograph, AX;
- einem Computertomograph, CT; und
einem Ultraschallgerät.

10. Verfahren nach einem der Ansprüche 1 bis 9, ferner umfassend die Schritte:
- Einlesen von weiteren Patientendaten, wobei die weiteren Patientendaten unabhängig von der Bilddatei erhoben worden sind;
- Extrahieren mindestens einer dritten Position einer anatomischen Struktur des Patienten aus den eingelesen weiteren Patientendaten;
- Vergleichen der aus den weiteren Patientendaten extrahierten mindestens einen dritten Position mit der ersten Position und/oder der mindestens einen zweiten Position; und
- Ausgeben eines Hinweises, falls der Vergleich eine Übereinstimmung ergibt, wobei der Hinweis gerichtet ist auf eine Überprüfung einer Konsistenz des Befunds mit den eingelesenen weiteren Patientendaten.

11. Verfahren nach einem der Ansprüche 1 bis 10, ferner umfassend die Schritte:
- Einlesen einer medizinischen Richtlinie, die mindestens für die Bilddatei, die eingelesenen (S102) Sensordaten und/oder die erste Position der anatomischen Struktur des Patienten als potentiell relevant eingestuft wird;
- Überprüfen des medizinischen Befunds hinsichtlich einer Konformität mit der medizinischen Richtlinie; und
- Ausgeben eines Warnsignals, wenn der medizinische Befund nicht konform mit der Richtlinie ist

12. Verfahren nach Anspruch 11, wobei der Schritt des Überprüfens des medizinischen Befunds hinsichtlich einer Konformität mit der medizinischen Richtlinie einen durch Maschinelles Lernen und/oder künstliche Intelligenz, KI, trainierten Algorithmus umfasst.

13. Vorrichtung (200) zur Sicherheitskontrolle eines medizinischen Befunds für einen Patienten basierend auf einer Bilddatei, die mittels eines bildgebenden medizinischen Geräts erfasst wurde, umfassend:
- Eine Sensorschnittstelle (202-1), die zum Empfangen von Sensordaten mindestens eines Sensors bezüglich der Bilddatei ausgebildet ist;
- Eine Zuordnungseinheit (204-1), die zum Zuordnen der eingelesenen Sensordaten zu einer ersten Position einer anatomischen Struktur des Patienten ausgebildet ist;
- Eine Extraktionseinheit (204-2), die zum Extrahieren mindestens einer zweiten Position einer anatomischen Struktur des Patienten aus dem medizinischen Befund ausgebildet ist;
- Eine Vergleichseinheit (204-3), die zum Vergleichen der ersten Position und der mindestens einen zweiten Position ausgebildet ist; und
- Eine Ausgabeschnittstelle (202-2), die zum Bereitstellen einer Ausgabe eines Warnsignals ausgebildet ist, falls der Vergleich keine Übereinstimmung ergibt.

14. Vorrichtung (200) nach Anspruch 13, wobei der mindestens eine Sensor ausgewählt ist aus der Gruppe bestehend aus:
- einem Bewegungssensor und/oder Eingabesensor einer Eingabeschnittstelle, wobei die Eingabeschnittstelle eine Computermaus, ein Trackpad, einen Trackball, einen Joystick, eine Tastatur, und/oder einen berührungsempfindlicher Bildschirm umfasst;
- einem Augen-Verfolgungssensor; und
- einem Zeitmesser, der dazu ausgebildet ist, die Zeitdauer der Betrachtung einer Schicht aus einem Bildstapel zu messen.

15. Vorrichtung (200) nach Anspruch 13 oder 14, die ferner dazu ausgebildet ist, die Schritte des Verfahrens gemäß Ansprüchen 2 bis 12 durchzuführen, und/oder Merkmale des Verfahrens gemäß den Ansprüchen 2 bis 12 zu umfassen.

16. System zur Sicherheitskontrolle eines medizinischen Befunds, umfassend:
- Mindestens einen Sensor zum Erfassen von Sensordaten bezüglich einer Bilddatei; und
- Eine Vorrichtung (200) gemäß einem der Vorrichtungsansprüche 14 bis 15, deren Sensorschnittstelle (202-1) die Sensordaten des mindestens einen Sensors einliest; und
- eine Warnsignalausgabeeinrichtung, die basierend auf des an der Ausgabeschnittstelle (202-2) der Vorrichtung (200) bereitgestellten Warnsignals das Warnsignal ausgibt.

17. Computerprogrammprodukt, umfassend Befehle, die bei der Ausführung des Programms durch einen Computer diesen veranlassen, das Verfahren nach einem der Ansprüche 1 bis 11 auszuführen.

18. Computerlesbares Speichermedium, umfassend Befehle, die bei der Ausführung durch einen Computer diesen veranlassen, das Verfahren nach einem der Ansprüche 1 bis 11 auszuführen.
